# EUROPEAN PATENT SPECIFICATION

(11) **EP 2 035 020 B1**
(45) Date of publication and mention of the grant of the patent: **17.11.2010**
(21) Application number: 07764311.2
(22) Date of filing: 20.06.2007
(51) Int. Cl.: A61K 33/24, C07F 15/00

(54) **PHARMACEUTICAL COMPOSITION FOR INJECTIONAL, PARTICULARLY TARGETED LOCAL ADMINISTRATION**
PHARMAZEUTISCHE ZUSAMMENSETZUNG ZUR INJEKTION, INSBESONDERE ZUR GEZIELTEN LOKALEN ANWENDUNG
COMPOSITION PHARMACEUTIQUE INJECTABLE, PARTICULIÈREMENT POUR ADMINISTRATION LOCALE CIBLÉE

(30) Priority: 20.06.2006 CZ 20060401
(43) Date of publication of application: 18.03.2009
(73) Proprietor: Pliva-Lachema A.S., 621 33 Brno (CZ)
(72) Inventor: FRANC, Ales, 638 00 Brno (CZ); SOVA, Petr, 500 02 Hradec Králové (CZ)
(74) Representative: Pavlica, Tomas
(86) International application number: PCT/CZ2007/000060
(87) International publication number: WO 2007/147373

(56) References cited:
- EP-A- 0 423 707
- EP-A- 0 812 852
- WO-A-2006/055352
- WO-A-2006/057998
- US-A- 4 845 124
- US-A1- 2003 109 487
- US-A1- 2004 156 888
- US-A1- 2006 099 146
- US-B1- 6 340 770

## Description

### Field of the Invention

The invention relates to a composition for injectional, particularly targeted local administration, enabling application of the active compound directly to the tumor site.

### Background of the Invention

Platinum complexes are generally known as effective substances having broad spectrum of antitumor effect and they are thus utilized for treating a number of tumor diseases. So far, the therapeutic practice has used only complexes of bivalent platinum, especially cisplatinum, carboplatinum or oxaliplatinum. Bivalent platinum complexes are unstable in the gastrointestinal tract and/or are very poorly absorbed. This makes the use of bivalent platinum complexes in an oral, otherwise for the patient more acceptable dosage form, impossible. It has been found that some complexes of tetravalent platinum have not this disadvantage and retain their antitumor activity even when administered orally. These complexes of tetravalent platinum were disclosed as novel chemical compounds for oral administration in EP 0 328 274, EP 0 423 707 and PCT/CZ99/00015 (published as WO 99/61451).

However, tetravalent platinum complexes exhibit very poor solubility in water (about 0.03 g/100 ml), low bulk density (about 0.2 g/ml), low tap density (about 0.4 g/ml), and a high electrostatic charge. The mentioned physical properties of the complexes represent an important problem in the preparation of their solid oral drug form. Moreover, complexes of tetravalent platinum are chemically unstable in contact with metals or with many currently used excipients. These problems are partially solved in PCT/CZ99/00015 which patent document describes the preparation of solid drug forms of specific tetravalent platinum complexes in the form of their inclusion complexes with cyclodextrins. According to the mentioned patent document, these inclusion complexes are obtained by reaction of cyclodextrins with tetravalent platinum complexes in an organic solvent and subsequent lyophilization, and are used for oral application. However, the employed amount of cyclodextrin markedly limits the content of the tetravalent platinum complex in the oral drug form, which represents a drawback. The obtained oral drug form thus has a relatively large volume and is difficult to swallow, making thus a single-dose oral application of greater amounts of tetravalent platinum complex impossible.

This disadvantage of oral forms of tetravalent platinum complexes could be eliminated by providing an injection form with sufficient content of tetravalent platinum complexes. From the state of the art it is evident that so far the preparation of such ah injection form has not been satisfactorily solved. The aim of this invention is thus to provide a pharmaceutical composition for injectional administration that would contain sufficient, therapeutically effective, amount of tetravalent platinum complexes.

### Summary of the Invention

Within the framework of the invention, it has been unexpectedly found that the below specified complex of tetravalent platinum, contained in the pharmaceutical composition according to the invention, can be used not only in systemic, but also in local administration, which makes it possible to apply the tetravalent platinum complex directly at the tumor site. Because of cumulation of the tetravalent platinum complex at the tumor site, a lower therapeutic dose of the complex can be used in comparison with a systemically applied dose of the same therapeutic effect. Moreover, the tetravalent platinum complex is thus protected against a longer circulation in the bloodstream and against chemical and enzymatic cleavage occurring in contact with biological protecting systems present in biological liquids, particularly in digestive juices and in blood, or during passage through the liver. In addition, use of a pharmaceutical composition according to the invention makes it possible to achieve a protracted effect of the tetravalent platinum complex.

This invention thus relates to a pharmaceutical composition for injectional, in particular targeted local administration, **characterized in that** it comprises a sterile suspension of platinum complex of structural formula II. 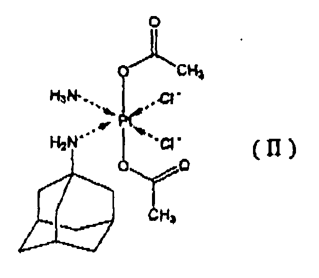 in a pharmaceutically acceptable hydrophilic or hydrophobic injection liquid phase, 100 % of particles of the platinum complex of structural formula II being of size smaller than 250 µm and 90 % of particles of the platinum complex of structural formula II being of size smaller than 10 µm.

Preferably, the weight ratio of the platinum complex of structural formula II to the liquid phase is 1:100 to 30:100, more preferably 5:100 to 10:100.

Preferably, as a liquid phase the pharmaceutical composition contains a hydrophilic liquid phase selected from the group consisting of water, glycerol and propylene glycol.

Preferably, the liquid phase contains at least one stabilizer, in particular a peptizer and/or a viscosifier.

Preferably, as peptizer, the liquid phase contains a polyol compound, advantageously selected from the group consisting of lactose, fructose, mannitol and sorbitol.

Preferably, the weight ratio of the polyol compound to the platinum complex of structural formula II is 0.1:1 to 10:1.

Preferably, as a viscosifier, the liquid phase contains a hydrophilic polymer, advantageously selected from the group consisting of polyvinylpyrrolidone, polyvinyl acetate, hydroxypropyl cellulose and hydroxypropyl methyl cellulose.

Preferably, the pharmaceutical composition contains 0.1 to 10 % by weight of at least one viscosifier, based on the total weight of the composition.

Preferably, as the liquid phase, the pharmaceutical composition contains a hydrophilic liquid phase which is an aqueous buffer pH 4 to 8.

Preferably, as the liquid phase, the pharmaceutical composition contains a hydrophobic liquid phase which is an oleophilic medium, selected in particular from the group consisting of oil for injections, olive oil and sunflower oil.

The invention also relates to the above mentioned pharmaceutical composition as a drug for use in the treatment of malignant tumors.

In the following part, the invention will be explained in more detail using specific examples of execution which are of illustrative nature only and do not limit in any way the scope of the invention that is unequivocally defined by the appending Claims.

In these Examples, the (OC-6-43)-bis(acetato)-(1-adamantylamine)-ammine-dichloroplatinum(IV) complex of structural formula II is denoted by its code name LA-12.

### Examples

### Example 1

### Preparation of hydrophilic suspension injection form of platinum complex LA-12

Mannitol (5 parts by weight) and hydroxypropyl methyl cellulose (1 part by weight) are dissolved in an aqueous buffer pH 4 (85 parts by weight) and the solution is sterilized by autoclaving. In the thus-obtained sterilized solution, platinum complex LA-12 (100 % of particles smaller than 250 µm) is aseptically suspended. The resulting suspension is aseptically filled into sterile vials.

### Example 2

### Preparation of hydrophobic injection form of platinum complex LA-12

Platinum complex LA-12, 100 % particles of which are of size smaller than 250 µm (5 parts by weight), is aseptically suspended in oil for injections (95 parts by weight). The resulting suspension is then aseptically filled into sterile vials.

## Claims

1. A pharmaceutical composition for injectional, in particular targeted local administration, **characterized in that** it comprises a sterile suspension of the (OC-6-43)-bis(acetato)-(1-adamantylamine)-ammine-dichloroplatinum (IV) complex of structural formula II 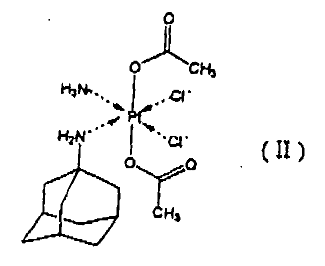 in a pharmaceutically acceptable hydrophilic or hydrophobic injection liquid phase, 100 % of particles of the platinum complex of structural formula II being of size smaller than 250 µm and
90 % of particles of the platinum complex of structural formula II being of size smaller than 10 µm.

2. The pharmaceutical composition according to claim 1, **characterized in that** the weight ratio of the platinum complex of structural formula II to the liquid phase is 1:100 to 30:100.

3. The pharmaceutical composition according to claim 2, **characterized in that** the weight ratio of the platinum complex of structural formula II to the liquid phase is 5:100 to 10:100.

4. The pharmaceutical composition according to claim 1, **characterized in that** as liquid phase it comprises a hydrophilic liquid phase selected from the group comprising water, glycerol and propylene glycol.

5. The pharmaceutical composition according to claim 1, **characterized in that** the liquid phase comprises at least one stabilizer, preferably peptizer and/or viscosifier.

6. The pharmaceutical composition according to claim 5, **characterized in that** as peptizer the liquid phase contains a polyol compound, preferably selected from the group comprising lactose, fructose, mannitol and sorbitol.

7. The pharmaceutical composition according to claim 6, **characterized in that** the weight ratio of the polyol compound to the platinum complex of structural formula II is 0.1:1 to 10:1.

8. The pharmaceutical composition according to claim 5, **characterized in that** as viscosifier the liquid phase contains a hydrophilic polymer, preferably selected from the group comprising polyvinylpyrrolidone, polyvinyl acetate, hydroxypropyl cellulose and hydroxypropyl methyl cellulose.

9. The pharmaceutical composition according to claim 8, **characterized in that** it contains 0.1 to 10 % by weight of at least one viscosifier, based on the total weight of the composition.

10. The pharmaceutical composition according to claim 1, **characterized in that** as a liquid phase it contains a hydrophilic liquid phase consisting of an aqueous buffer, pH 4 to 8.

11. The pharmaceutical composition according to claim 1, **characterized in that** as a liquid phase it contains a hydrophobic liquid phase consisting of an oleophilic medium, preferably selected from the group consisting of oil for injections, olive oil and sunflower oil.

12. A pharmaceutical composition according to any of the claims 1 to 11, as a drug for use in the treatment of malignant tumors.

## Patentansprüche

1. Eine pharmazeutische Zusammensetzung zur vornehmlich gezielten lokalen Injektionsverabreichung, **dadurch gekennzeichnet, dass** sie eine sterile Suspension des (OC-6-43)-Bis(azetato)-(1-adamantylamin)-ammine-dichloroplatin(IV)-Komplexes der strukturellen Formel II 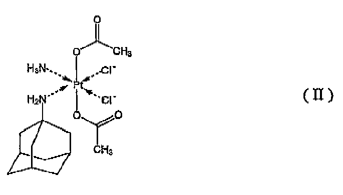 in einer pharmazeutisch akzeptablen, hydrophilen, flüssigen Injektionsphase beinhaltet, wobei 100% der Partikel des Platin-Komplexes der Strukturformel II von der Größe kleiner als 250 µm ist und 90% der Partikel des Platin-Komplexes der Strukturformel II von der Größe kleiner als 10 µm ist.

2. Die pharmazeutische Zusammensetzung nach Anspruch 1, **dadurch gekennzeichnet, dass** das Gewichtsverhältnis des Platin-Komplexes der Strukturformel II zur flüssigen Phase 1:100 bis 30:100 ist.

3. Die pharmazeutische Zusammensetzung nach Anspruch 2, **dadurch gekennzeichnet, dass** das Gewichtsverhältnis des Platinkomplexes der Strukturformel II zur flüssigen Phase 5:100 bis 10:100 ist.

4. Die pharmazeutische Zusammensetzung nach Anspruch 1, **dadurch gekennzeichnet, dass** sie als die flüssige Phase eine aus der aus Wasser, Glyzerol und Propylenglykol bestehender Gruppe ausgewählte hydrophile flüssige Phase beinhaltet.

5. Die pharmazeutische Zusammensetzung nach Anspruch 1, **dadurch gekennzeichnet, dass** die flüssige Phase mindestens einen Stabilisator, vorzugsweise ein Peptisationsmittel und/oder ein Viskosität steigendes Mittel beinhaltet.

6. Die pharmazeutische Zusammensetzung nach Anspruch 5, **dadurch gekennzeichnet, dass** die flüssige Phase als das Peptisationsmittel eine aus der vorzugsweise aus Laktose, Fruktose, Mannit und Sorbit bestehender Gruppe ausgewählte Polyolverbindung beinhaltet.

7. Die pharmazeutische Zusammensetzung nach Anspruch 6, **dadurch gekennzeichnet, dass** das Gewichtsverhältnis der Polyolverbindung zum Platin-Komplex der Strukturformel II 0,1:1 bis 10:1 ist.

8. Die pharmazeutische Zusammensetzung nach Anspruch 5, **dadurch gekennzeichnet, dass** die flüssige Phase als das Viskosität steigernde Mittel ein vorzugsweise aus der aus Polyvinylpyrrolidon, Polyvinylazetat, Flydroxypropylcellulose und, Hydroxypropylmethylcellulose bestehender Gruppe ausgewähltes hydrophiles Polymer beinhaltet.

9. Die pharmazeutische Zusammensetzung nach Anspruch 8, **dadurch gekennzeichnet, dass** sie 0,1 bis 10 Gew.-% von mindestens einem Viskosität steigernden Mittel, bezogen auf das Gesamtgewicht der Zusammensetzung, enthält.

10. Die pharmazeutische Zusammensetzung nach Anspruch 1, **dadurch gekennzeichnet, dass** sie als die flüssige Phase eine aus einem wässrigen pH 4-8 Puffer bestehende hydrophile flüssige Phase enthält.

11. Die pharmazeutische Zusammensetzung nach Anspruch 1, **dadurch gekennzeichnet, dass** sie als die flüssige Phase eine aus einem oleophilen Medium, vorzugsweise aus der aus Öl für Injektionszwecke, Olivenöl und Sonnenblumenöl bestehender Gruppe ausgewählte hydrophile flüssigen Phase enthält.

12. Die pharmazeutische Zusammensetzung nach einem der Ansprüche 1 bis 11, als ein Arzneimittel für den Einsatz in der Behandlung von malignen Tumoren.

## Revendications

1. Une composition pharmaceutique pour l'administration injectable particulierement pour l'administration à visée locale, **charactérisée en ce qu**'elle comprend une suspension sterile d'un complexe de (OC-6-43)-bis(acetato)-(1-adarnantylamine)-ammine-dichloroplatine (IV) de la formule structurale II 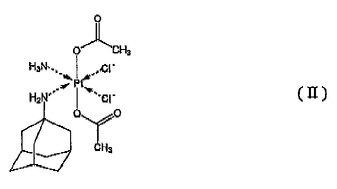 dans une phase liquide hydrophile ou hydrophobe injectable pharmaceutiquement acceptable, dont 100 % de particules du complex de platine de la formule structurale II ayant une taille inférieur à 250 µm et 90 % de particules du complex de platine de la formule structurale II ayant une taille inférieur à 10 µM.

2. La composition pharmaceutique selon la revendication 1 **caractérisée en ce que** le rapport en poids du complexe de platine de la formule structurale II à la phase liquide est de 1 : 100 à 30 : 100.

3. La composition pharmaceutique selon la revendication 2 **caractérisée en ce que** le rapport en poids du complexe de platine de la formule structurale II à la phase liquide est de 5 :100 à 10 : 100

4. La composition pharmaceutique selon la revendication 1 **caractérisée en ce qu'**elle comprend, en tant que phase liquide, une phase liquide hydrophile choisie du groupe consistant en eau, glycerol et propylenglycol.

5. La composition pharmaceutique selon la revendication 1 **caractérisée en ce que** la phase liquide comprend au moins un stabilisateur, de preference un agent de peptisation et/ou un agent modifiant la viscosité.

6. La composition pharmaceutique selon la revendication 5 **caractérisée en ce que** la phase liquide comprend, en tant qu'agent de peptisation, un composé polyol, de preference choisi du groupe consistant en lactose, fructose, mannitol et sorbitol.

7. La composition pharmaceutique selon la revendication 6 **caractérisée en ce que** le rapport en poids du composé de polyol au complexe de platine de la formule structurale II est de 0,1:1 à 10:1.

8. La composition pharmaceutique selon la revendication 5 **caractérisée en ce que** la phase liquide comprend, en tant qu'agent modifiant la viscosité, un polymer hydrophile, de preference choisi du groupe consistant en polyvinylpyrrolidone, polyvinylacetate, hydroxypropylcellulose et hydroxypropylmethylcellulose.

9. La composition pharmaceutique selon la revendication 8 **caractérisée en ce qu'**elle comprend de 0,1 à 10 % en poids d'au moins un agent modifiant la viscosité par rapport au poids total de la composition.

10. La composition pharmaceutique selon la revendication 1 **caractérisée en ce qu'**elle comprend, en tant que phase liquide, une phase liquide hydrophile consistant en solution tampon, pH 4 à 8.

11. La composition pharmaceutique selon la revendication 1 **caractérisée en ce qu'**elle comprend, en tant que phase liquide, une phase liquide hydrophobe consistant en médium oléophile, de preference choisi du groupe consistant en huile injectable, huille d'olive et huille de tourne-sol.

12. La composition pharmaceutique selon l'une quelconque des revendications 1 à 11 en tant que médicament pour l' utilisation dans le traitement des tumeurs malignes.
